# EUROPEAN PATENT APPLICATION

(11) **EP 1 535 590 A2**
(43) Date of publication of application: **01.06.2005**
(21) Application number: 04257385.7
(22) Date of filing: 29.11.2004
(51) Int. Cl.: A61F 9/00

(54) **Relocator**

(30) Priority: 27.11.2003 GB 0327548
(71) Applicant: Lensmate Limited, Skipton, North Yorkshire BD23 3LL (GB)
(72) Inventor: Duckworth, Brendan, Skipton, North Yorkshire BD23 3LL (GB)
(74) Representative: Stuttard, Garry Philip

(57) **Abstract**

The present invention relates to a contact lens relocator which may be used for fitting a contact lens to or removing a contact lens from a subject's eye.

## Description

The present invention relates to a contact lens relocator which may be used for fitting a contact lens to or removing a contact lens from a subject's eye.

Due to the sensitivity of the eye to foreign objects, there is a certain level of discomfort in fitting a contact lens to (or removing a contact lens from) the eye. For some subjects, the level of discomfort is acute and can be distressful. For example, it is common practice for contact lenses to be fitted to and removed from the eye using fingers. Normally for fitting a contact lens to the eye, the subject grips the lens between thumb and forefinger and brings the finger, thumb and eye together to adhere the lens to the surface of the cornea. For removing a contact lens from the eye, the subject grips the lens between thumb and forefinger and draws the lens off the surface of the cornea. Where the gripping action is against opposing edges, it may cause significant deformation of the lens which makes it difficult to fit or remove reliably or comfortably. Equally where the gripping action is bifacial, significant manual dexterity is required to manouevre the fingers with respect to the contact lens and surface of the cornea to allow the lens to adhere to the eye. Alternatively the subject may balance the contact lens on a finger and brings the finger and eye together to adhere the contact lens to the surface of the cornea. There is a risk that in bringing the finger and eye together the contact lens will be dislodged from the finger. It will also be appreciated that use of the fingers demands extreme cleanliness. FR2712398 of Brettes, US5785370 of Pomerantz, US4071272 of Drdlik, US6398277 of McDonald, US4238134 of Cointment, US3934914 of Carruthers, US5941583 of Raimondi and JP4138157 of Takuji all disclose simple mechanical devices for re-locating contact lenses by suction.

The present invention is based on the recognition that certain drawbacks of manual relocation of a contact lens may be overcome mechanically. More particularly, the present invention relates to a contact lens relocator comprising a remotely operable gripper which grips the contact lens mono-facially.

Thus viewed from a first aspect the present invention provides a contact lens relocator for relocating a contact lens from a first location to a second location comprising:
a gripper manipulable between a gripping mode in which the contact lens is gripped mono-facially in the first location and a non-gripping mode in which the contact lens is released in the second location;
a manipulator operatively connected to the gripper for manipulating the gripper remotely between the gripping mode and the non-gripping mode; and
an elongate connecting member connecting the manipulator to the gripper which extends along a path non-coincident with the axis of the gripper.

The contact lens relocator may be advantageously used for fitting a contact lens to a subject's eye. In other words, the first location may be remote from the eye (eg a storage or sterilising solution) and the second location may be the eye. Equally the contact lens relocator may be used for removing a contact lens from a subject's eye. In other words, the second location may be remote from the eye (eg a storage or sterilising solution) and the first location may be the eye. By gripping the contact lens mono-facially, the contact lens is substantially undistorted in the gripping mode and is therefore fitted in (or removed from) the eye reliably, hygienically and with minimal discomfort. By allows the contact lens to be fitted to or removed from the eye with less discomfort for the subject as the hand which holds the relocator is away from the direct line of sight of the eye. By being non-coincident with the axis of the gripper, the elongate connecting member advantageously allows the contact lens to be fitted to or removed from the eye with less discomfort for the subject as the hand of the subject is away from the direct line of sight.

Preferably in the gripping mode the gripper grips solely the exterior face of the contact lens. For this purpose, the gripper preferably includes a gripping surface which substantially conforms configurationally to at least a part of the substantially convex exterior surface of the contact lens. For example, the gripping surface may be substantially concave (*eg* cup-shaped).

The gripper may include a gripping surface which substantially conforms dimensionally to at least a part of the substantially convex exterior surface of the contact lens. Typically the gripping surface is smaller than the exterior surface of the contact lens. One or more of the edges of the gripping surface may be rounded. This reduces any tendency for the gripping surface to damage the surface of the eye or the contact lens. For example, the gripping surface may conveniently be substantially circular. The gripping surface is generally of a material which is soft, is physiologically tolerable to the eye, conforms with health and safety requirements and exhibits longevity. For example, the material may be silicon or neoprene.

Preferably in the gripping mode the gripper grips the contact lens (*eg* grips the exterior surface of the contact lens) pneumatically or hydraulically.

Preferably in the non-gripping mode the gripper releases the contact lens (*eg* releases the exterior surface of the contact lens) pneumatically or hydraulically.

For gripping the exterior surface of the contact lens mono-facially, the gripping surface may be in fluid communication with the manipulator. Preferably the elongate connecting member defines a bore and the manipulator comprises:
a chamber containing a fluid reservoir which is in fluid communication with the gripping surface through the bore. The elongate connecting member is connected (preferably substantially sealingly connected) at a first end to the manipulator and is connected (preferably substantially sealingly connected) at a second end to the gripper.

The manipulator may be manipulated (*eg* squeezed) to drive fluid from the fluid reservoir so as to manipulate the gripper into the non-gripping mode. The manipulator may be squeezed and then released so as to manipulate the gripper into the gripping mode. For this purpose, the material of the body which defines the chamber is resilient. Preferably the chamber is manually deformable (*eg* squeezable). By way of example, the chamber may be a pipette body (*eg* a pipette bulb).

The manipulator may manipulate the gripper into the gripping mode such that there is a residual amount of fluid in the chamber. Preferably the manipulator is discretely manipulable to a first extent whereat the residual amount of fluid remains in the chamber. The first extent may be the extent to which the manipulator is squeezable under an average force or under a force applied in a first direction. For this purpose, the manipulator is typically internally configured to withstand total collapse under the average force. Preferably the manipulator is discretely manipulable to a second extent whereat at least a proportion of the residual amount of fluid is driven from the chamber. The second extent maybe the extent to which the manipulator is squeezable under a greater than average force or under a force applied in a second direction.

In a preferred embodiment, the manipulator is non-uniformly manipulable. Preferably, the manipulator is stepwise manipulable. Preferably the manipulator comprises a first internal surface and a second internal surface, wherein when the manipulator is unmanipulated the first internal surface and the second internal surface are spaced apart and when the manipulator is manipulated to a first extent the first internal surface and second internal surface contact. Preferably the first internal surface and second internal surface are a diametrically opposed first internal surface and second internal surface respectively. Particularly preferably the first internal surface and second internal surface are a diametrically opposed first internal surface and second internal surface respectively of an internal annular rib.

Preferably the manipulator defines a substantially bulbous chamber. Typically the bulbous chamber extends into an elongate neck. The elongate neck may define externally a gripping position for manipulation of the manipulator. The internal annular rib may be coincident internally with the gripping position.

The elongate connecting member may be linear or non-linear. Preferably the elongate connecting member is at least partly (preferably fully) curved. Particularly preferably the elongate connecting member is partly curved at least at or near to the gripper.

The elongate connecting member may be a tube (*eg* a cylindrical tube). Preferably the first end of the bore terminates in an aperture in the gripping surface (*eg* substantially centrally in the gripping surface).

Preferably the elongate connecting member is at least partially compressible (*eg* shock absorbent). A compressible elongate connecting member may advantageously absorb any impact of the elongate connecting member on the eye during relocation of a contact lens thereby preventing damage to the eye and the lens and eliminating any discomfort.

For this purpose, the elongate connecting member may be compositionally or mechanically compressible. Typically the elongate connecting member is resilient. For example, the elongate connecting member may be non-linear. Preferably, the elongate connecting member is curved. A non-linear elongate connecting member may incorporate regularly or irregularly one or more discontinuities (*eg* radial and/or axial discontinuities) which compress on contact with the eye to allow a cushioning effect. The one or more discontinuities may be one or (preferably) more than one of the group selected from loop, spiral, constriction or distension. Preferably the elongate connecting member adopts at least partially a helical, concertina-like or bellow-like configuration.

In a preferred embodiment, the elongate connecting member incorporates one or more (preferably a plurality of) axially compressible loops.

In a preferred embodiment, the elongate connecting member incorporates one or more (preferably a plurality of) axially compressible, truncated bi-conic sections. Preferably adjacent truncated bi-conic sections have a common truncated apex. Particularly preferably a plurality of truncated bi-conic sections are successively interconnected at a common truncated apex.

On the elongate connecting member (*eg* at or near to the first end) may be mounted a sleeve which serves to improve rigidity. The sleeve may be enveloped by a collar. Where the manipulator comprises a chamber containing a fluid reservoir, the collar may bear in a substantially airtight manner against an interior wall of the chamber. For this purpose, the interior wall of the chamber may be provided with a continuous flange and the collar bears in a substantially airtight manner against the continuous flange.

The gripper may comprise a pad. The pad may be fitted (*eg* in a substantially air tight manner) to the second end of the elongate connecting member. In a preferred embodiment, the pad comprises: a tubular main body defining a bore extending outwardly at a first end into a flexible skirt and outwardly at a second end into a sleeve, wherein the exterior surface of the flexible skirt is the gripping surface and the sleeve is mounted (*eg* in a substantially air tight manner) to the second end of the elongate connecting member. For this purpose, the sleeve may be largely cylindrical.

The edge of the flexible skirt may be rounded. The first end of the bore may terminate in an aperture substantially centrally in the exterior surface of the flexible skirt. The exterior surface of the flexible skirt conforms (dimensionally and configurationally) to the substantially convex exterior surface of a contact lens.

The manipulator and gripper may be integral (*eg* a moulded single part) or separate parts.

In a preferred embodiment, the contact lens relocator further comprises:
a container for containing the gripper,
wherein a portion of the manipulator engages (preferably sealingly engages) at least a part of the container such as to contain the gripper.

The part of the container is typically the lid. For example, where the manipulator comprises a chamber containing a fluid reservoir, an end wall of the lid may incorporate a retaining aperture and the chamber may be retained in the retaining aperture.

Preferably the chamber extends radially outwardly at its open end into a radial lip upon which the inner edge of the end wall defining the aperture is seated. Particularly preferably a radial flange extends outwardly from the exterior surface of the chamber and is spaced closely apart from and substantially parallel to the radial lip so that the edge of the end wall defining the aperture is captured therebetween.

Alternatively preferably the chamber extends into an elongate neck with one or more ribs (preferably external ribs) for engaging (*eg* threadedly or resiliently engaging) complementary ribs on the end (preferably interior end) of the container. Preferably the chamber extends into an elongate neck with one or more ribs (preferably internal ribs) for engaging (*eg* threadedly or resiliently engaging) complementary ribs on the end (preferably interior end) of the elongate connecting chamber.

The container can usefully be filled with storage solutions such as sterilising solutions to keep the gripper sterile and ready for use.

Viewed from a further aspect the present invention provides a contact lens relocator for relocating a contact lens from a first location to a second location comprising:
a gripper manipulable between a gripping mode in which the contact lens is gripped mono-facially in the first location and a non-gripping mode in which the contact lens is released in the second location;
a manipulator operatively connected to the gripper for manipulating the gripper remotely between the gripping mode and the non-gripping mode, wherein the manipulator comprises a chamber containing a fluid reservoir; and
an elongate connecting member connecting the manipulator to the gripper, wherein the elongate connecting member defines a bore and the manipulator is in fluid communication with the gripping surface through the bore and wherein the manipulator is adapted to be discretely manipulable to a first extent whereat a residual amount of fluid remains in the chamber.

Viewed from a yet further aspect the present invention provides a method for relocating a contact lens from a first location to a second location comprising:
obtaining a contact lens relocator comprising:
   a gripper manipulable between a gripping mode and a non-gripping mode; and
   a manipulator operatively connected to the gripper for manipulating the gripper between the gripping mode and the non-gripping mode;
firstly manipulating the manipulator;
gripping the contact lens in the first location;
relocating the relocator and contact lens to the second location;
secondly manipulating the manipulator; and
releasing the contact lens in the second location.

Preferably in the gripping mode the contact lens is gripped mono-facially.

Firstly manipulating the manipulator and gripping the contact lens in the first location preferably comprises:
squeezing (*eg* manually squeezing) the manipulator from a first configuration to a second configuration;
applying the gripper in the non-gripping mode to the exterior surface of the contact lens in the first location whilst the manipulator is in the second configuration;
releasing (*eg* manually releasing) the manipulator from the second configuration to a third configuration to manipulate the gripper to the gripping mode.

Secondly manipulating the manipulator preferably comprises:
squeezing (*eg* manually squeezing) the manipulator from the third configuration to a fourth configuration. The manipulator may then be released from the fourth configuration to the first configuration.

For fitting a contact lens, the second location is the eye and the first location may be a storage container and/or sterilising solution. For removing a contact lens, the first location is the eye and the second location may be a storage container and/or sterilising solution.

Preferably the contact lens relocator is as hereinbefore defined.

The present invention will now be described in a non-limitative sense with reference to the accompanying drawings in which:
Figure 1 illustrates in cross-section a first embodiment of the present invention;
Figure 2 illustrates a bottle for use with the first embodiment of figure 1;
Figure 3 illustrates a partial close up view of the first embodiment of the present invention in use;
Figure 4 illustrates partially a second embodiment of the present invention;
Figure 5 illustrates partially a third embodiment of the present invention;
Figure 6 illustrates a fourth embodiment of the present invention in longitudinal cross section;

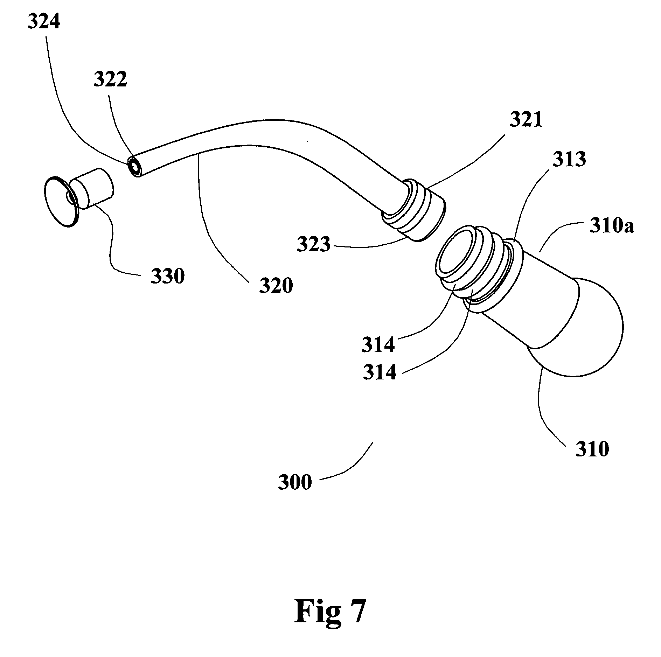

Figure 7 is an exploded view of the fourth embodiment of the present invention without the lid;

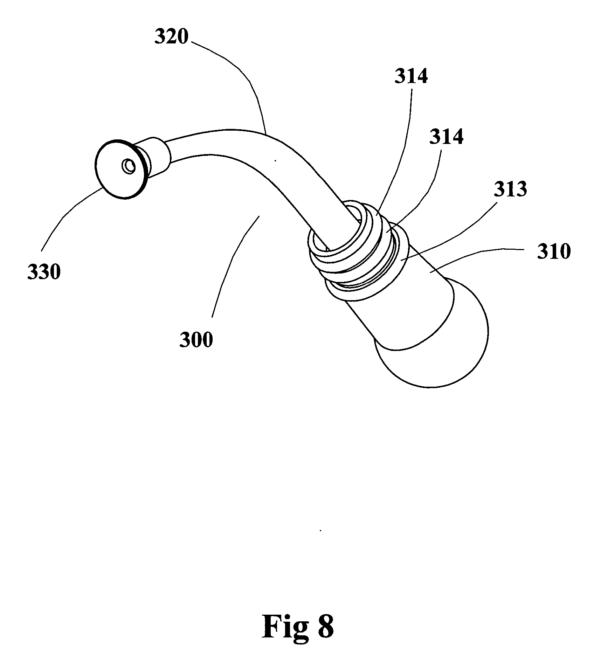

Figure 8 is an isometric view of the fourth embodiment of the present invention without the lid; and

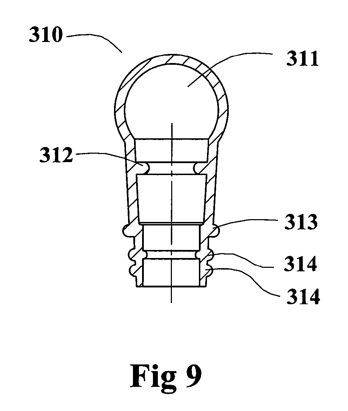

Figure 9 is a longitudinal cross section of the pipette bulb of the fourth embodiment.

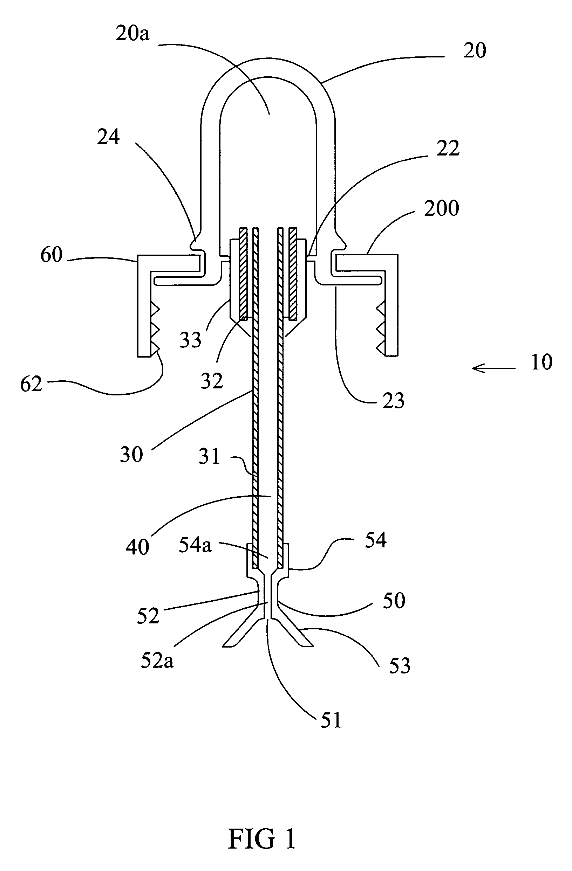

Figure 1 illustrates a first embodiment of the contact lens relocator of the present invention designated generally by reference numeral (10). The relocator (10) comprises at its distal end a resilient pipette bulb (20) connected to a stalk (30) which includes a hollow stalk tube (31). The pipette bulb (20) defines a largely bulbous chamber (20a) and extends radially outwardly at its open end into a radial lip (23). A radial flange (24) extends outwardly from the exterior surface of the pipette bulb (20) and is spaced closely apart from and substantially parallel to the radial lip (23). The hollow stalk tube (31) defines a bore (40) which provides fluid communication between the chamber (20a) of the pipette bulb (20) and the exterior surface of a pad (50) connected to the hollow stalk tube (31) at its proximal end.

The pad (50) comprises a suction tube (52) defining a narrow bore (52a) extending outwardly at a first end into a flexible skirt (53) and outwardly at a second end into a sleeve (54) itself defining a wider bore (54a). The edge of the flexible skirt (53) is round. The first end of the narrow bore (52a) terminates in a suction hole (51) substantially centrally in the exterior concave face of the flexible skirt (53). The exterior concave face of the flexible skirt (53) serves to accommodate the convex outer surface of a contact lens. The sleeve (54) fits over the proximal end of the hollow stalk tube (31) to form a substantially airtight seal.

The stalk (30) further includes a tubular collar (32) at its distal end which fits over the hollow stalk tube (31) and provides rigidity. A tubular sleeve (33) envelopes the tubular collar (32) and provides a substantially airtight seal between the tubular collar (32) and the hollow stalk tube (31) at the proximal end of the tubular collar (32).

A continuous flange (22) is formed on the interior wall of the pipette bulb (20). The distal end of the stalk (30) fits into the pipette bulb (20) such that a substantially airtight seal is formed between the continuous flange (22) and the tubular sleeve (33). This together with the substantially airtight seal between the sleeve (54) and hollow stalk tube (31) ensures that fluid communication between the bulbous chamber (20a) of the pipette bulb (20) and the environment is via the suction hole (51) only.

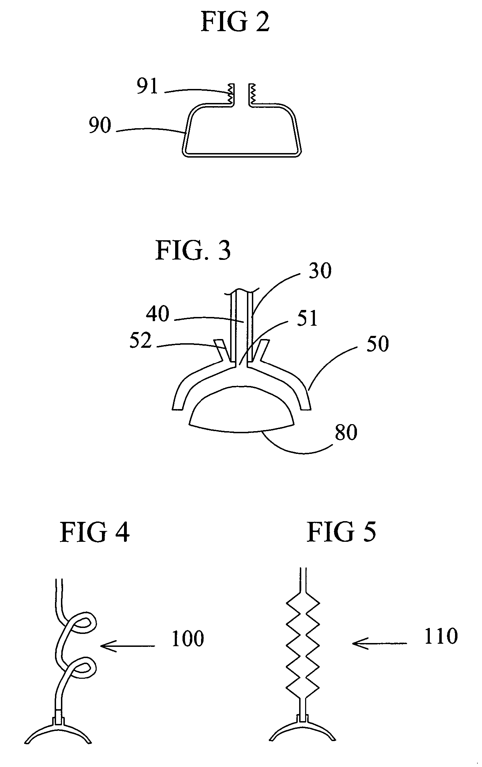

The pipette bulb (20) is held within an aperture in a bottle lid (60). To keep the bottle lid (60) firmly in this position, the end wall (200) of the bottle lid (60) defining the aperture is captured between the radial lip (23) and radial flange (24) on the pipette bulb (20). As the bottle lid (60) is screwed onto an appropriately shaped bottle neck (91) of a bottle (90 - see Figure 2) and approaches the end of a screw thread (62), the bottle neck (91) presses on the radial lip (23) causing the radial lip (23) to form a substantially airtight seal with the bottle lid (60). The relocator (10) can be stored in the bottle (90) when it is not in use. For this purpose, the bottle (90) may be substantially filled with a sterilising solution that will sterilise the pad (50) while it is being stored.

Figure 3 shows use of the relocator (10) in a preferred embodiment. The bore (40) and chamber (20a) of the pipette bulb (20) are normally filled with fluid of which a substantial part may be liquid such as a sterilising solution (which is physiologically tolerable in the eye). To insert a contact lens (80) in the eye, the pipette bulb (20) is manually squeezed to eject an amount of fluid through the suction hole (51). Whilst the squeezing pressure on the pipette bulb (20) is maintained, the relocator (10) is moved so that the pad (50) is gently touching the exterior face of the free contact lens and the suction hole (51) is located substantially centrally thereon. The pipette bulb (20) is then manually released at least partially and the resilient expansion of the pipette bulb (20) lowers the pressure in its chamber (20a) and at the suction hole (51) causing a net suction force sufficient to grip the contact lens (80) on to the pad (50).

With the contact lens (80) gripped in this way, the relocator (10) is moved freely and the contact lens (80) will not detatch from the pad (50) until the pipette bulb (20) is squeezed again. Thus the contact lens (80) is carefully positioned in the eye with the relocator (10) and when it is located in the correct position the pipette bulb (20) can be squeezed and the suction force on the contact lens (80) is released. This separates the contact lens (80) from the relocator (10) and the relocator (10) is removed leaving the contact lens (80) fitted to the eye.

Removing the contact lens (80) from the eye is achieved by using generally the same steps as those described above for fitting the contact lens. More specifically, the pipette bulb (20) is manually squeezed and the suction hole (51) is then located substantially in the centre of the exterior face of the contact lens (80) whilst manual pressure is maintained. The pipette bulb (20) is then released at least partially which causes a net suction force sufficient to grip the contact lens (80) on to the pad (50). The relocator (10) and gripped contact lens (80) are then removed from the eye. Once the contact lens (80) has been moved to a suitable storage location the pipette bulb (20) is squeezed and the suction force on the contact lens (80) is released. This separates the contact lens (80) from the relocator (10) and the contact lens (80) is left in its storage location.

Figure 4 shows a preferred embodiment of the relocator (10) which includes a helical hollow stalk tube (100). In all other respects the relocator (10) is the same as that described above for the first embodiment. The shape defined by the outer surface of the stalk tube (100) is helical with a plurality of loops which compress on contact with the eye to allow a cushioning effect during use.

Figure 5 shows a preferred embodiment of the relocator (10) which includes a concertina-like hollow stalk tube (110). In all other respects the relocator (10) is the same as that described above for the first embodiment. The shape defined by the outer surface of the stalk tube (110) is analogous to that of concertina bellows *ie* a series of alternating large and small circles coincident on the same axis and separated at regular intervals in the axial direction. The circles are joined by truncated conic sections. This concertina-like hollow stalk tube (110) compresses on contact with the eye to allow a cushioning effect during use.

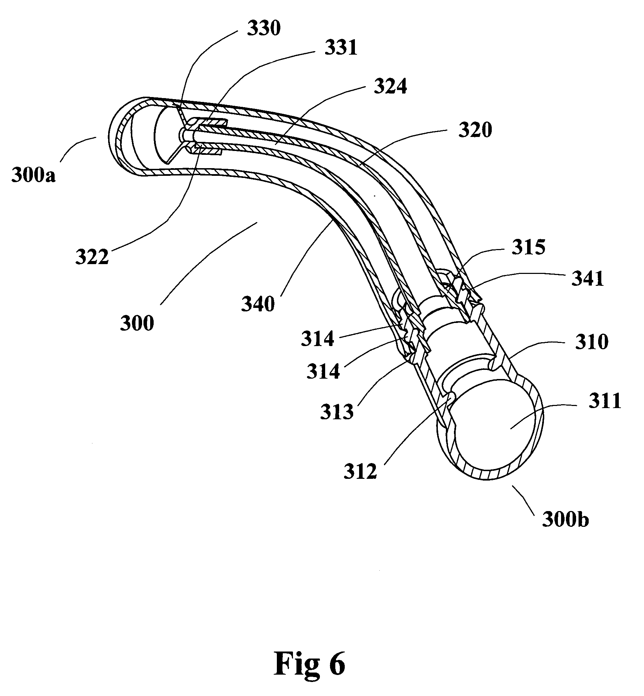

Figure 6 shows a fourth embodiment of a contact lens relocator (300) according to the present invention. The relocator (300) has a proximal end (300a) and a distal end (300b) and generally comprises a pipette bulb (310) defining a largely bulbous resilient chamber (311), a curved stalk (320) defining a hollow bore (324), a pad (330) and a lid (340). The hollow bore (324) provides fluid communication between the chamber (311) and the exterior of the pad (330). The pipette bulb (310) is connected at its proximal end to the distal end of the stalk (320). The stalk (320) is connected at its proximal end (322) to the pad (330) and has at its distal end a cuff (323).

The pipette bulb (310) extends into an elongate neck (310a) with an inner rib (312) and an outer stop rib (313). The neck (310a) terminates in a series of outer connecting ribs (314) and an inner connecting rib (315). The resilient chamber (311) is filled with fluid which may be expelled by applying a force to the outside of the pipette bulb (310). When an average force is applied to the pipette bulb (310), diametrically opposed portions of the inner rib (312) come into contact before the resilient bulb (310) is fully collapsed so that the inner rib (312) prevents all the fluid from being expelled. This ensures that there will be available residual fluid that may be expelled (*eg* by a greater than average force) to straightforwardly release the contact lens from the pad (330).

The distal end of the lid (340) fits over the stalk (320) to engage the pipette bulb (310) at its proximal end to form a sealed enclosure around the stalk (320) and the pad (330). The outer stop rib (313) is designed to delimit the engagement of the lid (340) and the pipette bulb (310). The outer connecting ribs (314) engage complementary inner connecting ribs (341) of the lid (340) to form the seal. The inner connecting rib (315) engages a cuff groove (321) on the cuff (323) of the stalk (320) to form an airtight seal.

The pad (330) is largely identical to that described hereinbefore in respect of the first embodiment. The pad (330) has a flexible sleeve (331) which engages the proximal end (322) of the stalk (320).

## Claims

1. A contact lens relocator for relocating a contact lens from a first location to a second location comprising:
a gripper manipulable between a gripping mode in which the contact lens is gripped mono-facially in the first location and a non-gripping mode in which the contact lens is released in the second location;
a manipulator operatively connected to the gripper for manipulating the gripper remotely between the gripping mode and the non-gripping mode; and
an elongate connecting member connecting the manipulator to the gripper which extends along a path non-coincident with the axis of the gripper.

2. A relocator as claimed in claim 1 wherein in the gripping mode the gripper grips solely the exterior face of the contact lens.

3. A relocator as claimed in claim 2 wherein the gripper includes a gripping surface which substantially conforms configurationally to at least a part of the substantially convex exterior surface of the contact lens.

4. A relocator as claimed in claim 2 wherein the gripper includes a gripping surface which substantially conforms dimensionally to at least a part of the substantially convex exterior surface of the contact lens.

5. A relocator as claimed in claim 1 wherein the elongate connecting member defines a bore and the manipulator comprises:
a chamber containing a fluid reservoir which is in fluid communication with the gripping surface through the bore.

6. A relocator as claimed in claim 5 wherein the manipulator is discretely manipulable to a first extent whereat a residual amount of fluid remains in the chamber.

7. A relocator as claimed in claim 6 wherein the first extent is the extent to which the manipulator is squeezable under an average force or under a force applied in a first direction.

8. A relocator as claimed in claim 6 or 7 wherein the manipulator is discretely manipulable to a second extent whereat at least a proportion of the residual amount of fluid is driven from the chamber.

9. A relocator as claimed in claim 6, 7 or 8 wherein the second extent is the extent to which the manipulator is squeezable under a greater than average force or under a force applied in a second direction wherein the second direction is different to the first direction.

10. A relocator as claimed in any preceding claim wherein the manipulator is non-uniformly manipulable.

11. A relocator as claimed in any of claims 6 to 10 wherein the manipulator comprises:
a first internal surface and
a second internal surface,
wherein when the manipulator is unmanipulated the first internal surface and the second internal surface are spaced apart and when the manipulator is manipulated to a first extent the first internal surface and second internal surface contact.

12. A relocator as claimed in claim 11 wherein the first internal surface and second internal surface are a diametrically opposed first internal surface and second internal surface respectively of an internal annular rib.

13. A relocator as claimed in claim 12 wherein the manipulator defines externally a gripping position for manipulation of the manipulator and the internal annular rib is coincident internally with the gripping position.

14. A relocator as claimed in any preceding claim wherein the elongate connecting member is at least partly curved.

15. A relocator as claimed in any preceding claim wherein the elongate connecting member is compositionally or mechanically compressible.

16. A relocator as claimed in any preceding claim further comprising:
a container for containing the gripper,
wherein a portion of the manipulator engages at least a part of the container such as to contain the gripper.

17. A contact lens relocator for relocating a contact lens from a first location to a second location comprising:
a gripper manipulable between a gripping mode in which the contact lens is gripped mono-facially in the first location and a non-gripping mode in which the contact lens is released in the second location;
a manipulator operatively connected to the gripper for manipulating the gripper remotely between the gripping mode and the non-gripping mode, wherein the manipulator comprises a chamber containing a fluid reservoir; and
an elongate connecting member connecting the manipulator to the gripper, wherein the elongate connecting member defines a bore and the manipulator is in fluid communication with the gripping surface through the bore and wherein the manipulator is adapted to be discretely manipulable to a first extent whereat a residual amount of fluid remains in the chamber.

18. A relocator as claimed in claim 17 wherein the first extent is the extent to which the manipulator is squeezable under an average force or under a force applied in a first direction.

19. A relocator as claimed in claim 17 or 18 wherein the manipulator is discretely manipulable to a second extent whereat at least a proportion of the residual amount of fluid is driven from the chamber.

20. A relocator as claimed in claim 17, 18 or 19 wherein the second extent is the extent to which the manipulator is squeezable under a greater than average force or under a force applied in a second direction.

21. A relocator as claimed in any of claims 17 to 20 wherein the manipulator is non-uniformly manipulable.

22. A relocator as claimed in any of claims 6 to 10 wherein the manipulator comprises:
a first internal surface and
a second internal surface,
wherein when the manipulator is unmanipulated the first internal surface and the second internal surface are spaced apart and when the manipulator is manipulated to a first extent the first internal surface and second internal surface contact.

23. A relocator as claimed in claim 22 wherein the first internal surface and second internal surface are a diametrically opposed first internal surface and second internal surface respectively of an internal annular rib.

24. A relocator as claimed in claim 23 wherein the manipulator defines externally a gripping position for manipulation of the manipulator and the internal annular rib is coincident internally with the gripping position.

25. A method for relocating a contact lens from a first location to a second location comprising:
obtaining a contact lens relocator comprising:
a gripper manipulable between a gripping mode and a non-gripping mode; and
a manipulator operatively connected to the gripper for manipulating the gripper between the gripping mode and the non-gripping mode;
firstly manipulating the manipulator;
gripping the contact lens in the first location;
relocating the relocator and contact lens to the second location;
secondly manipulating the manipulator; and
releasing the contact lens in the second location.
